# EUROPEAN PATENT APPLICATION

(11) **EP 4 505 999 A1**
(43) Date of publication of application: **12.02.2025**
(21) Application number: 23306345.2
(22) Date of filing: 07.08.2023
(51) Int. Cl.: A61K 9/50, A61P 25/08, A61K 31/195

(54) **PROBENECID FORMULATIONS**

(71) Applicant: Panntherapi, 30000 Nîmes (FR)
(72) Inventor: BRILLAUD, Elsa, 30000 Nîmes (FR); GUILLERMIN, Alexandra, 30000 Nîmes (FR)
(74) Representative: Icosa

(57) **Abstract**

The present invention relates to a core-shell particulate formulation presenting a core comprising probenecid in an amount ranging from 10% to 70% in weight relative to the total weight of the core, in association with at least one pharmaceutically acceptable excipient, wherein the core is coated with a shell coating composition comprising triethyl citrate, a mineral charge, polyvinyl acetate, polyvinylpyrrolidone and sodium lauryl sulfate, characterized in that the weight ratio of polyvinyl acetate on polyvinylpyrrolidone ranges from 1.5 to 4.

## Description

### FIELD OF INVENTION

The present invention relates to modified release pharmaceutical formulations of probenecid and in particular pediatric probenecid formulations. The invention further relates to such formulations for their use in the treatment of epilepsy.

### BACKGROUND OF INVENTION

Probenecid is a benzoic acid derivative with an excellent safety profile that was developed in the 1950's to decrease the renal tubular excretion of penicillin; and has been used to increase the serum concentration of several antibiotics and antivirals. During the initial studies using probenecid (referred to as Benemid), probenecid was observed to have a strong uricosuric effect and quickly became the standard of treatment of gout. It was found to decrease uric acid levels in the serum via inhibition of organic acid reabsorption, such as uric acid, by the renal proximal tube by acting as a competitive inhibitor of the organic anion transporters (OATs) and thus preventing OAT-mediated reuptake of uric acid from the urine to the serum. Even though probenecid has a minimal adverse effect profile, its clinical use has declined significantly as other therapies for gout have shown improved efficacy.

As disclosed in the international application WO2019/012109, probenecid is also suitable for treating epilepsy in a subject in need thereof, such as for example in pediatric subjects.

Considering that the half-life of probenecid is limited and dose-dependent, based on the currently available probenecid formulations, treating epilepsy would necessitate the administration of 4 intakes per day. Furthermore, probenecid is a highly lipophile molecule. Thus, there is a need to supply a high-charge probenecid formulation that is suitable for the probenecid's physicochemical properties and that shall ensure the patients' compliance by limiting the number of intakes per day to up to two intakes per day.

Furthermore, given the specificities of the pediatric subjects, and in particular the limited volume of excipients to be administered, the high charge probenecid formulation needs to enable the minimal possible amount of excipients. In addition, pediatric formulations need to be easy to ingest, and advantageously compatible with food or drink compositions, in order to ensure the pediatric patients' compliance.

In addition, pediatric probenecid formulations need to be compliant with good manufacturing processes, present sufficient stability during shelf-life and present a good reproducibility among the produced batches.

The present invention, addresses the above needs by supplying a modified release formulation of probenecid as disclosed herein. In particular the present formulation can present up to 50% w/w probenecid charge, despite the highly lipophile profile of probenecid as well as a prolonged liberation of probenecid, thereby limiting the number of medication intakes to up to twice a day. Advantageously, this can be achieved by enabling a quantitatively and qualitatively limited amount of excipients. More advantageously, the formulation is compatible with food ingredients and can be dispersed into food or drink compositions in order to ensure the subject's, in particular the pediatric subject's, compliance. Furthermore, the liberation of probenecid is compliant to the therapeutic purposes of epilepsy treatment and the manufacturing process of the present formulations allows a reproducible quality profile among the produced batches that present a sufficient shelf-life stability.

### SUMMARY

The present invention relates to a core-shell particulate formulation, wherein the core comprises probenecid in an amount ranging from 10% to 70% in weight relative to the total weight of the core, in association with at least one pharmaceutically acceptable excipient. In said core-shell particulate formulation, the core is coated with a shell coating composition comprising triethyl citrate, a mineral charge, polyvinyl acetate, polyvinylpyrrolidone and sodium lauryl sulfate, characterized in that the weight ratio of polyvinyl acetate on polyvinylpyrrolidone ranges from 1.5 to 4.

In some embodiments, the shell coating composition comprises in weight relative to the total weight of the core-shell particulate formulation:
- from 0.4% to 1.0%, preferably from 0.5% to 0.8% of triethyl citrate,
- from 8.0% to 16%, preferably from 10% to 12% of polyvinyl acetate,
- from 2.0% to 6.0%, preferably from 3.0% to 4.5% polyvinylpyrrolidone,
- from 0.05% to 0.2% of sodium lauryl sulfate, and
- from 1.0% to 6.0%, more preferably from 3.0% to 5.0% of a mineral charge, even more preferably the mineral charge is talc.

The second coating composition may represent from 2% to 30% preferably from 10% to 30%, more preferably from 15% to 25%, typically about 20% relative to the total weight of the core-shell particulate formulation.

The core may be a mixture of probenecid with at least one pharmaceutically acceptable excipient. Alternatively, the core may be a particle of an inert core consisting of at least one pharmaceutically acceptable excipient that is coated with a first coating composition comprising probenecid.

According to some embodiments, the at least one pharmaceutically acceptable excipient can be selected from cellulose, microcrystalline cellulose, cellulose derivatives, starch, modified starch, dextran maltodextrin sucrose, lactose, mannitol, mannitol, sorbitol, maltitol, trehalose, calcium carbonate, magnesium carbonate, and silica.

The formulation may optionally further comprise a final coating in an amount ranging from 2 to 20% relative to the total weight of the core-shell particulate formulation. For instance, such final coating may comprise:
- polyvinyl alcohol in association with talc, titanium dioxide, glyceryl mono and dicaprylocaprate and sodium lauryl sulfate;
- polyvinyl alcohol-polyethylene glycol graft copolymer, polyvinyl alcohol, and silicone dioxide;
- hydroxypropylmethylcellulose (Hypromellose) and talc; or
- a mixture thereof.

According to some embodiments where the core consists of a pharmaceutically acceptable inert core that is coated with a first coating composition, said first coating composition may comprise:
- from 10% to 70% w/w of probenecid;
- from 20% to 40% w/w of hydroxypropylcellulose;
- from 1% to 3% w/w of a surfactant; and
- optionally an anti-foaming agent;
   in weight relative to the total weight of the coated core.

According to some indicative embodiments, the surfactant is a non-ionic surfactant, preferably the surfactant is polyoxyethylene (20) sorbitan monooleate.

Furthermore, such inert core may consist of microspheres consisting of at least one pharmaceutically acceptable excipient as described above that may present an average diameter ranging from 100 µm to 5 mm. According to some embodiments, the inert core consists of microcrystalline cellulose microspheres presenting an average diameter ranging from 100 µm to 800 µm, more preferably from 300 to 400 µm, the average diameter being determined with the sieving method.

According to some embodiments where the core consists of a pharmaceutically acceptable inert core that is coated with a first coating composition, the first coating composition may represent from 50% to 80% in weight relative to the total dry weight of the coated inert core.

Optionally, the cores or coated inert cores may further comprise a seal-coating composition in an amount ranging from 2% to 20% in weight relative to coated cores comprising, said seal-coating composition comprising:
- polyvinyl alcohol in association with talc, titanium dioxide, glyceryl mono and dicaprylocaprate and sodium lauryl sulfate;
- polyvinyl alcohol-polyethylene glycol graft copolymer, polyvinyl alcohol, and silicone dioxide;
- hydroxypropylmethylcellulose (Hypromellose) and talc; or
- a mixture thereof.

According to a further aspect, the invention relates to the formulation according to the invention for use as a drug. According to a specific embodiment, the formulation may be for use in the treatment of epilepsy.

Lastly, the invention relates to a process for preparing the core-shell particulate formulation to the invention, comprising the steps of:
a) Supplying a composition of cores comprising probenecid in an amount ranging from 10% to 70% in weight relative to the total weight of the core, in association with at least one pharmaceutically acceptable excipient,
b) Coating the cores with a shell coating composition comprising triethyl citrate, a mineral charge, polyvinyl acetate, polyvinylpyrrolidone and sodium lauryl sulfate, characterized in that the weight ratio of polyvinyl acetate on polyvinylpyrrolidone ranges from 1.5 to 4, preferably by spray drying, leading to a core-shell particulate formulation,
c) Optionally further coating with a final coating as described above;
d) Recovering the core-shell particulate formulation obtained in any one of steps b) or c).

According to the variant where the cores are coated inert cores, step a) may comprise the steps:
a1) Supplying a composition of inert core consisting of at least one pharmaceutically acceptable excipient;
a2) Coating the microcrystalline cellulose microspheres, preferably by spray drying, with a first coating composition, thereby supplying coated cores of microcrystalline cellulose, wherein the first coating composition comprises, in weight relative to the total weight of the coated cores:
   - from 10% to 70% w/w of probenecid;
   - from 20% to 40% w/w of hydroxypropylcellulose;
   - from 1% to 3% w/w of a surfactant; and
   - optionally an anti-foaming agent;
a3) optionally, further coating the coated cores of microcrystalline cellulose with a seal coating composition as described above,
a4) recovering the particulate formulation obtained in step a2) or in step a3).

### DETAILED DESCRIPTION

This invention relates to modified release formulations of probenecid that present a high charge of such active pharmaceutical ingredient, that present a sufficient stability during shelf-life and that are particularly suitably for pediatric subjects.

### MR formulation

The invention thus relates to a core-shell probenecid formulation wherein the core is a formulation that comprises probenecid in an amount ranging from 10% to 70% in weight relative to the total weight of the core, in association with at least one pharmaceutically acceptable excipient, said core being is coated with a coating composition (herein referred to as shell or second coating composition) as described herein below.

The second or shell coating composition comprises triethyl citrate, a mineral charge, polyvinyl acetate, polyvinylpyrrolidone and sodium lauryl sulfate, wherein the weight ratio of polyvinyl acetate on polyvinylpyrrolidone ranges from 1.5 to 4, preferably from 2.5 to 3.5. In some embodiments, the weight ratio of polyvinyl acetate on polyvinylpyrrolidone ranges from 3.0 to 3.5, typically about 3.3. In some other embodiments, the weight ratio of polyvinyl acetate on polyvinylpyrrolidone ranges from 2.5 to less than 3.0, or preferably is about 2.6. In the context of the present invention, "about" preceding a figure means plus or less 10%, typically plus or less 5% of the value of said figure.

The second coating may be in an amount sufficient to modulate the liberation of probenecid.

Probenecid refers to the active pharmaceutical ingredient compound having the CAS number 57-66-9 and presenting the structure of formula (I):

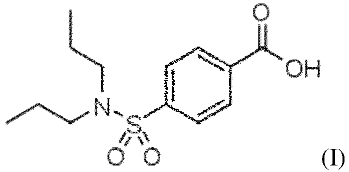

Probenecid encompasses free-base probenecid (sometimes also referred to as 4-(dipropylsulfamoyl)benzoic acid, 4-[(dipropylamino)sulfonyl]-benzoic acid or PBN), as well as pharmaceutically acceptable salts thereof (4-(dipropylsulfamoyl)benzoate salts or 4-[(dipropylamino)sulfonyl]-benzoate salts). Also encompassed are prodrugs, isomers, and polymorphs of probenecid. Probenecid can exist in unsolvated forms as well as solvated forms, including hydrated forms. In general, the solvated forms are equivalent to unsolvated forms and are encompassed within the scope of the present invention. Probenecid may exist in multiple crystalline or amorphous forms. In general, all physical forms are equivalent for the uses contemplated by the present invention and are intended to be within the scope of the present invention.

According to some embodiments, the probenecid is in the form of particles, typically crystals, wherein, at least 70 % preferably at least 90% or 92% of the probenecid particles are smaller than 75 µm, as determined by the sieve method. Alternatively, the probenecid is in the form of particles, typically crystals, wherein according to the laser diffraction method, the d(0,9) is about 150 µm.

According to some other embodiments, the probenecid particles may present a d(0,9) of less than 40 µm according to the laser diffraction method, that can be herein characterized as micronized probenecid. For instance, the probenecid particles may present a d(0,9) of 21 µm, a d(0,5) of 8 µm and a d(0,1) of 2,2 µm according to the laser diffraction method.

In some embodiments, the second or shell coating composition represents from 2% to 30%, preferably from 10% to 30%, more preferably from 15% to 25%, typically about 20% relative to the total weight of the core-shell particulate formulation.

In some embodiments, the second composition comprises in weight relative to the total weight of the core-shell particulate formulation:
- from 0.4% to 1.0%, preferably from 0.5% to 0.8% of triethyl citrate,
- from 8.0% to 16%, preferably from 10% to 12% of polyvinyl acetate,
- from 2.0% to 6.0%, preferably from 3.0% to 5.0% polyvinylpyrrolidone,
- from 0.05% to 0.2% of sodium lauryl sulfate, and
- from 1.0% to 6.0%, more preferably from 3.0% to 5.0% of a mineral charge.

In some embodiments, the second composition comprises in weight relative to the total dry weight of the shell coating composition:
- from 2.0% to 5.0%, preferably from 2.5% to 4.0% of triethyl citrate as a plasticizer,
- from 40.0% to 82.0%, preferably from 50.0% to 60.0% of polyvinyl acetate as a coating polymer,
- from 10% to 30.0%, preferably from 15% to 25.0% of polyvinylpyrrolidone as a pore-forming agent,
- from 0.25% to 1.0 %, of sodium lauryl sulfate, and
- from 5.0% to 30.0%, more preferably from 15.0% to 25.0% of a mineral charge.

The mineral charge can act as a filler and/or as an opacifying agent. Any mineral charge known in the art that is can be pharmaceutically acceptable can be formulated in the present formulation such as for example talc, calcium carbonate, calcium chloride etc. In some embodiments, the mineral charge is talc.

The binder of the second coating composition is polyvinyl acetate (PVAc) exerting the coating function of the second or shell coating composition. Polyvinylpyrrolidone (PVP), acting as a pore-forming agent in the PVAc coating layer is also added in order to ensure an efficient probenecid dissolution and absorption by the subject that ingests the present formulation. Without willing to be bound by a theory, the presence of PVAc and PVP in the presently claimed ratios ensure not only the protection of the probenecid that is present in the first coating composition but also ensure its timely release in the upper gastrointestinal tract where the active absorption of probenecid takes place thereby enhancing the treatment efficacy.

In some embodiments, compositions comprising PVAc, PVP and sodium lauryl sulfate are readily available in the market such as for example Kollicoat ^{®} SR 30 D, wherein the PVAc/PVP ratio is about 10. However, in that case, in order to ensure the PVAc/PVP weight ratio of the invention, an additional source of PVP needs to be used such as for example PVP K30^{®}. Alternatively, it is in the purview of the skilled artisan to prepare the second coating composition of the invention by mixing separate sources of PVAc, PVP and sodium lauryl sulfate by enabling any commercial source of such excipients.

The coating of the immediate release particles (or cores) with the second (shell) composition may be carried out by any means known in the art such as for example by a spray-drying process as described herein below.

It is of note that the present modified release core-shell particles present a sufficient stability during shelf-life. Optionally, the core-shell particles as herein described may present an additional coating, herein designated as final-coating, that coats the shell coating. In some embodiments, the final coating may be present in an amount ranging from 2% to 20%, preferably from 10% to 20% in weight relative to the core-shell formulation. The final-coating composition may comprise:
- a composition comprising Polyvinyl alcohol in association with talc, titanium dioxide, glyceryl mono and dicaprylocaprate and sodium lauryl sulfate, such as for example the commercially available Opadry^{®} AMB composition
- a composition comprising polyvinyl alcohol-polyethylene glycol graft copolymer, typically in an amount ranging from 55% to 65%, polyvinyl alcohol, typically in an amount ranging from 35% to 45%, and silicon dioxide, typically in an amount ranging from 0.1% to 0.3%, the percentages being in weight relative to the total weight of the final-coating composition. An exemplary embodiment of such final coating composition is the commercially available Kollicoat^{®} Protect composition,
- a composition comprising Hypromellose and talc, such as for example the commercially available Opadry^{®} 03A69 composition; or
- a mixture thereof.

### The Core

The core is a formulation, according to any formulation in the purview of the skilled artisan, also referred to as an immediate release formulation, that comprises probenecid in an amount ranging from 10% to 70%, from 20% to 60%, or from 25% to 50%, in weight relative to the total weight of the core. Such core formulation may also be characterized herein as an immediate release formulation of probenecid.

In some embodiments, the at least one pharmaceutically acceptable excipient is selected from cellulose, microcrystalline cellulose, cellulose derivatives such as hydroxycellulose, hydroxypropylcellulose or hydroxypropylmethylcellulose, starch, modified starch such as hydrolyzed starch, dextran, maltodextrin, sucrose, lactose, mannitol, sorbitol, maltitol, trehalose, calcium carbonate, magnesium carbonate, and silica. In some embodiments, the at least one pharmaceutically acceptable excipient is selected from cellulose, microcrystalline cellulose, hydroxypropylcellulose, hydroxypropylmethylcellulose, preferably microcrystalline cellulose.

According a first variant, the core is a mixture of probenecid with at least one pharmaceutically acceptable excipient. For instance, probenecid may be mixed with at least one pharmaceutically acceptable excipient, such as for example as described herein above, and then be compressed into a core.

According to a second variant, the core is a particle of an inert core consisting of at least one pharmaceutically acceptable excipient, such as for example as described herein above, that is coated with a first coating composition comprising probenecid. The coating according to the second variant can be for example a direct coating with probenecid, i.e. the probenecid compound being adsorbed or absorbed on the surface of the inert core. Alternatively, probenecid can be mixed with at least one pharmaceutically acceptable excipient to form a coating composition (herein referred to as a first coating composition) that coats the inert cores.

According to such variant, the inert core may consist of microspheres consisting of at least one pharmaceutically acceptable excipient, as described above, presenting an average diameter ranging from 100 µm to 5 mm, from 100 µm to 2 mm, from 100 µm to 1000 µm, preferably from 100 µm to 800 µm, more preferably from 200 µm to 600 µm, such as from 300 µm to 400µm.

According to some exemplary embodiments, the core is a particle of an inert core consisting of Microcrystalline cellulose (MCC) that is coated with a first coating composition comprising probenecid thereby forming coated cores of MCC with the first coating composition. The first coating composition comprises in weight relative to the total weight of the at least one coated core:
- probenecid in an amount ranging from 10 to 70%, preferably from 20 % to 50%, more preferably from 30% to 50%, typically about 40%;
- a binder, in particular hydroxypropylcellulose in an amount ranging from 20 to 40%, preferably from 25% to 35%, typically about 30%,
- a surfactant in an amount ranging from 1 to 3%, preferably from 1.5% to 2.5%, typically about 2.0%, and
- optionally an anti-foaming agent.

According to an alternative description of the first coating composition, it can be described by expressing the amounts of the excipients as relative to the amount of the active pharmaceutical ingredient, probenecid. Accordingly, the first coating composition may comprise, in weight relative to the total weight of the comprised probenecid:
- A binder, in particular hydroxypropylcellulose, in an amount ranging from 55 to 85%, preferably from 60% to 80%, more preferably from 70% to 80% typically about 75%,
- a surfactant in an amount ranging from 2 to 8%, preferably from 3% to 6%, typically about 5%, and
- optionally an anti-foaming agent.

Microcrystalline cellulose (MCC) is a natural polymer composed of glucose units joined by a 1-4 beta glycosidic bond. Preferably, the MCC microspheres present and average diameter ranging from, 100 µm to 800 µm, 300 µm to 400 µm, typically about 350 µm, as determined by the sieve method, such as for example Cellets^{®} 350.

The selection of HPC as binder according to the exemplary embodiments not only the high charge of the formulation particles with probenecid, but also allowed a repeatable production of particles with a reliable dosage of the formulation in probenecid, i.e. wherein the amount of the probenecid in the first coating composition is quantitively charged on the MCC microspheres, with no substantial losses of probenecid during coating.

Hydroxypropylcellulose (HPC) is non-ionic cellulose ether, that is typically used in pharmaceutical compositions as a binder. Typically, the molar substitution degree of the glucose monomers by hydroxypropyl moieties ranges from 2.0 to 4.0. In some embodiments, the HPC presents a hydroxypropyl substitution ranging from more than 14%, more than 15% or more than 20% in weight relative to total weight of the HPC composition. For instance, the HPC used in the first composition coating may be Pharmacoat^{®} 606.

The first coating composition according to some exemplary embodiments also comprises a surfactant, that may typically act as a wetting agent that, without willing to be bound by a theory, facilitates the homogenous dispersion of probenecid within the first coating composition and as a consequence on the coated inert cores, typically the MCC microspheres. According to some embodiments, the surfactant is a non-ionic surfactant in an amount ranging from 0.5 to 8%, preferably from 1% to 5%, typically about 2% in weight relative to the total weight of the at least one core. According to some specific embodiments, the surfactant is Polyoxyethylene (20) sorbitan monooleate, also known as Tween 80^{®}.

The coating of the MCC microsphere cores may be carried out by any means known in the art such as for example by a spray-drying process as described herein below. In order to facilitate the coating procedure, the first coating composition may further comprise an antifoaming agent in an amount ranging from 0.02% to 0.2% relative to the total weight of the coated cores or from 0.05 to 0.5 % in weight relative to the total weight of the comprised probenecid. According to some embodiments, the anti-foaming agent is simethicone (CAS n° 8050-81-5).

The amount of coating or coating percentage may define the amount of the probenecid that is charged onto the formulation. The amount or the percentage of coating can be determined by any means known in the art such as for example by comparing the weight of the obtained coated inert cores to the weight of the used inert cores. In some embodiments, the first coating composition represents from 50% to 90%, preferably from 65% to 75%, typically about 75% in weight relative to the total dry weight of the coated core.

Optionally, the immediate release particles as herein described may present an additional coating, herein designated as seal-coating.

The seal-coating composition, when present, may be in an amount sufficient to impermeabilize the immediate release particulate formulation of the invention. In some embodiments, the seal coating is in an amount ranging from 2% to 20%, preferably from 10% to 20% in weight relative to the particulate formulation of the cores according to the first variant or the coated inert cores of the second variant.

Indicatively, the seal-coating composition may comprise:
- a composition comprising Polyvinyl alcohol in association with talc, titanium dioxide, glyceryl mono and dicaprylocaprate and sodium lauryl sulfate, such as for example the commercially available Opadry^{®} AMB composition
- a composition comprising polyvinyl alcohol-polyethylene glycol graft copolymer, typically in an amount ranging from 55% to 65%, polyvinyl alcohol, typically in an amount ranging from 35% to 45%, and silicone dioxide, typically in an amount ranging from 0.1% to 0.3%, the percentages being in weight relative to the total weight of the final-coating composition. An exemplary embodiment of such final coating composition is the commercially available Kollicoat^{®} Protect composition,
- a composition comprising Hydroxypropylmethylcellulose and talc, such as for example the commercially available Opadry^{®} 03A69 composition; or
- a mixture thereof.

Hydroxypropylmethylcellulose (HPMC), also known as hypromellose is non-ionic cellulose ether, typically made from natural cotton fiber under series of chemical processing by etherification of the cellulose moieties of the cellulose backbone structure having the CAS n° 9004-65-3. It is an odorless, tasteless and non-toxic white powder that can be dissolved in cold water to form a transparent viscous solution with the gelling and/or thickening properties. HPMC can typically present a methoxy substitution of the hydroxyl moieties of the cellulose backbone in an amount ranging from 15.0% to 30.0%, typically from 19.0% to 30.0%, preferably from 27.0% to 30.0% or from 19.0% to 27.0%, in weight relative to total weight of the HPMC composition.

In view of the above, the core-shell particulate formulation can be summarized as a formulation comprising:
- A core comprising probenecid in an amount ranging from 10% to 60% in weight relative to the total weight of the core, in association with at least one pharmaceutically acceptable excipient; According to the second variant of the invention, the core, also referred to as an inert core, is coated with a first coating composition, such as for example according to the exemplary embodiments described above
- Optionally, the core may further comprise a release coating, coating the core or the first coating of the inert core according to the second variant,
- A second, or shell, coating composition as described above, and
- Optionally a final coating composition as described above.

### Pharmaceutical composition and uses

The invention further relates to a pharmaceutical composition comprising the immediate or the modified release formulation of the invention as such or in association with at least one pharmaceutically acceptable excipient that is in the purview of the skilled artisan such as for example at least one excipient selected from bulking agents, taste or smell enhancing agents, and flow-improving agents.

According to another aspect, the invention relates to the formulation or the pharmaceutical composition of the invention for its use as a drug. The invention also relates to the use of the formulation or the pharmaceutical composition of the invention for the manufacture of a drug.

In some indicative embodiments, the drug is for use in the treatment of a disease selected from gout, hyperuricemia and epilepsy. According to some preferred embodiments, the drug is for use in the treatment of epilepsy.

The invention also relates to a method for treating a disease selected from gout, hyperuricemia and epilepsy, comprising administering to a subject in need thereof a therapeutically effective amount of the formulation or the pharmaceutical composition of the invention.

As used herein, the term **"subject"** refers to an animal, preferably a warm-blooded animal, more preferably a mammal, even more preferably a human. In one embodiment, a subject may be a mammal. Mammals include, but are not limited to, all primates (human and non-human), cattle (including cows), horses, pigs, sheep, goats, dogs, and cats. In one embodiment, the subject is a human. In one embodiment, the subject is a patient, *i.e.,* is awaiting the receipt of, or is receiving medical care or was/is/will be the object of a medical procedure, or is monitored for the development of an epileptic disease, disorder or condition. In one embodiment, the subject is an adult (for example a subject above the age of 18). In another embodiment, the subject is a child (for example a subject below the age of 18). In one embodiment, the subject is a male. In another embodiment, the subject is a female.

According to some embodiments, the subject may be a **substantially healthy subject.** In the case of epilepsy, a substantially healthy subject is a subject who has not been previously diagnosed or identified as having or suffering from an epileptic disease, disorder or condition. In one embodiment, a substantially healthy subject shows no onset of an epileptic disease, disorder or condition, *i.e.,* the subject has not yet acquired, developed, or first experienced epileptic seizures.

As used herein, the terms **"treating"** or **"treatment"** or **"alleviation"** refers to both therapeutic treatment and prophylactic or preventative measures; wherein the object is to prevent or slow down (lessen) the targeted pathologic condition or disorder, such as for example an epileptic disease, disorder or condition. Those in need of treatment include those already with the disease, disorder or condition as well as those prone to have the disease, disorder or condition, or those in whom the disease, disorder or condition is to be prevented. A subject is successfully **"treated"** for a specific disease, disorder or condition, such as for example an epileptic disease, disorder or condition if, after receiving a therapeutic amount of probenecid according to the present invention, the subject shows observable and/or measurable reduction in one or more of the followings: epileptic seizures, in particular clinical epileptic seizures (that may be completely absent); reduced morbidity and mortality; improvement in quality-of-life issues. The above parameters for assessing successful treatment and improvement in the disease, disorder or condition are readily measurable by routine procedures familiar to a physician.

As used herein, the terms **"therapeutically effective amount"** mean level or amount of agent that is aimed at, without causing significant negative or adverse side effects to the target, (1) delaying or preventing the onset of a target disease, disorder, or condition; (2) slowing down or stopping the progression, aggravation, or deterioration of one or more symptoms of the target disease, disorder, or condition; (3) bringing about ameliorations of the symptoms of the target disease, disorder, or condition; (4) reducing the severity or incidence of the target disease, disorder, or condition; or (5) curing the target disease, disorder, or condition. A therapeutically effective amount may be administered prior to the onset of the target disease, disorder, or condition, for a prophylactic or preventive action. Alternatively or additionally, the therapeutically effective amount may be administered after initiation of the target disease, disorder, or condition, for a therapeutic action. The determination of the therapeutically effective amount can be determined by the physician. For instance, the therapeutically effective amount of probenecid for the treatment of epilepsy in pediatric subjects can range from 10 to 50 mg of probenecid per kg of the subject per day.

### Process

The invention also relates to a process for preparing the core-shell particulate formulation of the invention.

According to some embodiments the process comprises the steps of:
a) Supplying a composition of cores comprising probenecid in an amount ranging from 10% to 70% in weight relative to the total weight of the core, in association with at least one pharmaceutically acceptable excipient, as described above. It is of note that the cores may be in any one of the two variants of the core as described above, i.e According the first variant, the core is a mixture of probenecid with at least one pharmaceutically acceptable excipient. For instance, probenecid may be mixed with at least one pharmaceutically acceptable excipient, such as for example as described herein above, and then be compressed into a core. Whereas according to the second variant, the core is a particle of an inert core consisting of at least one pharmaceutically acceptable excipient, as described herein above, that is coated with a first coating composition comprising or consisting of probenecid;
b) Coating the cores with a shell coating composition comprising triethyl citrate, a mineral charge, polyvinyl acetate, polyvinylpyrrolidone and sodium lauryl sulfate, characterized in that the weight ratio of polyvinyl acetate on polyvinylpyrrolidone ranges from 1.5 to 4, leading to a core-shell particulate formulation.

The coating step b) can be carried out by any means in the purview of the skilled artisan. For example, the cores can be coated by spray drying e.g. by spray drying on a fluid-bed machine.

The core-shell particulate formulation can be recovered after the coating step b). However, the process may further comprise:
c) Optionally coating the particulate formulation of step b) with a final coating, as described above;

Thus, according to some optional embodiments, the core-shell particulate formulation can be obtained in any one of steps b) or c).

As set above, the core is a formulation, according to any formulation in the purview of the skilled artisan, that comprises probenecid in an amount ranging from 10% to 70% in weight relative to the total weight of the core, as detailed above. When referring to the second variant of the invention, step a) can be indicatively further described as comprising:
a1) Supplying a composition of inert core consisting of at least one pharmaceutically acceptable excipient, such as for example MCC inert cores,
a2) Coating the inert cores, preferably by spray drying, with a first coating composition, thereby supplying coated cores of the inert cores, wherein the first coating composition comprises, in weight relative to the total weight of the coated cores:
   - from 10% to 60% w/w of probenecid;
   - from 20% to 40% w/w of hydroxypropylcellulose;
   - from 1% to 3% w/w of a surfactant; and
   - optionally an anti-foaming agent;
a3) optionally, further coating the coated cores of microcrystalline cellulose with a seal coating composition as described above, and
a4) recovering the particulate formulation obtained in step a2) or in step a3).

### BRIEF DESCRIPTION OF THE DRAWINGS

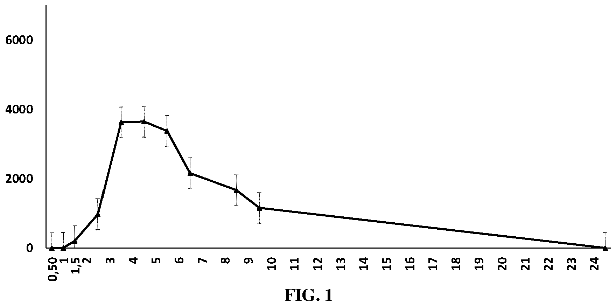

**Figure 1** is a graph showing the in vivo exposure level to probenecid (plasmatic concentration in ng/mL) over time (h) after the administration of the MR2 formulation according to the invention.

### EXAMPLES

The present invention is further illustrated by the following examples.

### Example 1: Preparation of exemplary immediate-release cores

### Materials and Methods

Immediate-release cores were prepared according to table 1.

**Table 1: Indicative IR core of probenecid**

| IR formulation - cores | | | |
|---|---|---|---|
| | Function | Ingredient | Quantity % (w/w) |
| A | Inert core | Microcrystalline cellulose (Cellet ^{®} 350) | 27.9 |
| B | API | Probenecid | 40.0 |
| C | Binder | HPC (Klucel^{®} EF Pharm) | 30.0 |
| D | Surfactant | Tween 80 | 2.0 |
| E | Antifoam agent | simethicone | 0.08 |

Ingredients B-E were suspended in water by mixing with Ultraturrax^{®}, thereby supplying a first coating composition

The microcrystalline cellulose inert cores were then coated by fluid-bed spray-drying with the fluid bed coater Glatt GPCG1 at a bottom spray configuration.

The obtained immediate release formulation was then assessed on the probenecid (API) in vitro dissolution at pH 6.8 with paddles rotating at 50 rotations per minute.

### Results

The immediate release formulation presented insignificant amount of agglomerates and with a satisfying yield of 89.1%.

The immediate-release formulation presented a liberation of probenecid in the dissolution medium of at least 80% in about 2.5 hours.

### Example 2: Preparation of modified -release core-shell formulation of the invention

### Materials and Methods

The immediate-release coated cores of Example 1 were used as cores of the core shell formulation of the invention.

Two modified-release core-shell formulations were prepared according to the composition of table 2 hereinbelow.

The immediate-release coated cores of Example 1 were coated by fluid-bed spray-drying with the fluid bed coater Glatt GPCG1 at a bottom spray configuration using the second coating composition presented in table 2.

**Table 2: Modified release formulations MR1 and MR2 according to the invention.**

| Modified release formulation: | | MR1 | MR2 |
|---|---|---|---|
| Function | Ingredient | Quantity % (w/w) | |
| IR release bearing the API | Immediate release cores of Example 1 | 80.0 | 80.0 |

| Second coating composition: | | | |
|---|---|---|---|
| Plasticizer | Triethyl citrate | 0.6 | 0.6 |
| Pore forming agent | PVP | 2.3 | 3.1 |
| (Coating mix) | Kollicoat^{®} SR30D* comprising: | (12.8) | (12.2) |
| | PVAc | 11.5 | 11.0 |
| | PVPₖ | 1.2 | 1.1 |
| | Sodium lauryl sulfate | 0.13 | 0.12 |
| Anti-tacking agent | talc | 4.3 | 4.1 |
| | Total PVP | 3.5 | 4.2 |
| | Weight ratio of PVAc/total PVP | 3.3 | 2.6 |

| | | | |
|---|---|---|---|
| ^{∗}Kollicoat^{®} SR30D is a commercially available coating mix of an aqueous dispersion of polyvinyl acetate (PVAc:27%), sodium lauryl sulfate (0.3%) and inherently comprising PVP (hereinabove indexed as PVPₖ : 2.7%). The dry subject matter of Kollicoat SR30D, e.g. after spray drying, comprises about 90% PVAc, about 9% PVPₖ and about 10% sodium lauryl sulfate. Total PVP consists in the sum of the exogenously added PVP and the PVPk comprised in the Kollicat^{®} mixture. | | | |

The obtained modified release formulations were then assessed on the probenecid (API) in vitro dissolution at pH 6.8 with paddles rotating at 50 rotations per minute according to Ph. Eur 2.9.3. (01/2023:20903).

### Results

The controlled release core-shell particles presented a homogenous shell coating and their dissolution was as follows:
- MR1: 80% dissolution of probenecid was achieved by 20h of the dissolution assay;
- MR2: 80% dissolution of probenecid was achieved by 7 hours, and a dissolution of more than 90% was achieved by 9 hours.

### Example 3: In vivo liberation of probenecid with a formulation according to the invention

Formulations IR (of example 1) and MR2 (of example 2) were orally administered to minipigs (2g probenecid/minipig) during a single administration and cross over study into two groups of subjects (IR and MR2).

With a focus on the dosage of the plasmatic unbound probenecid, that is responsible for the biological effects, the pharmacokinetic properties are presented in table 3 for the IR formulation and table 4 for the core-shell formulation according to the invention, MR2.

**Table 3. The Pharmacokinetic parameters of unbound Probenecid in mini pig plasma samples -Group IR**

| Group IR | t_{1/2} | Tₘₐₓ | Cₘₐₓ | MRT |
|---|---|---|---|---|
| | (h) | (h) | (ng/mL) | (h) |
| Mean | 1.19 | 1.25 | 11806 | 4.10 |
| SD | 0.40 | 0.35 | 7208 | 0.80 |

**Table 4. The Pharmacokinetic parameters of unbound Probenecid in mini pig plasma samples - Group MR2**

| Group MR2 | t_{1/2} | Tₘₐₓ | Cₘₐₓ | MRT |
|---|---|---|---|---|
| | (h) | (h) | (ng/mL) | (h) |
| Mean | 2.80 | 3.667 | 5063 | 6.58 |
| SD | 1.46 | 0.58 | 2378 | 1.60 |

Interestingly, the formulation MR2 according to the invention increased the half-life (t_{1/2}) by a factor of 2.3 for the unbound probenecid. It is of note that, the unbound form of probenecid is the form that shall bind to the pharmacological targets exert its pharmacological effects.

Still more interestingly, the formulation allows to enhance the mean residency time (MRT) by a factor of 1.7, so that when the minipig pharmacokinetic evidence is transposed to humans, a twice-a-day administration of the formulation shall supply therapeutically effective amounts of probenecid in the plasma. Indeed, as illustrated in Figure 1, the administration of MR2 formulation according to the invention lead to an exposure level sufficient for a subject to be treated with an intake of up to two intakes of the formulation according to the invention per day.

## Claims

1. A core-shell particulate formulation, wherein the core comprises probenecid in an amount ranging from 10% to 70% in weight relative to the total weight of the core, in association with at least one pharmaceutically acceptable excipient, said core being coated with a shell coating composition comprising triethyl citrate, a mineral charge, polyvinyl acetate, polyvinylpyrrolidone and sodium lauryl sulfate, **characterized in that** the weight ratio of polyvinyl acetate on polyvinylpyrrolidone ranges from 1.5 to 4.

2. The formulation according to claim **1,** comprising in weight relative to the total weight of the core-shell particulate formulation:
- from 0.4% to 1.0%, preferably from 0.5% to 0.8% of triethyl citrate,
- from 8.0% to 16%, preferably from 10% to 12% of polyvinyl acetate,
- from 2.0% to 6.0%, preferably from 3.0% to 4.5% polyvinylpyrrolidone,
- from 0.05% to 0.2% of sodium lauryl sulfate, and
- from 1.0% to 6.0%, more preferably from 3.0% to 5.0% of a mineral charge, even more preferably the mineral charge is talc.

3. The formulation according to claim **1** or claim **2,** wherein the core is a mixture of probenecid with at least one pharmaceutically acceptable excipient or wherein the core is a particle of an inert core consisting of at least one pharmaceutically acceptable excipient that is coated with a first coating composition comprising probenecid.

4. The formulation according to any one of claims **1** to **3,** wherein the at least one pharmaceutically acceptable excipient is selected from cellulose, microcrystalline cellulose, cellulose derivatives, starch, modified starch, dextran maltodextrin sucrose, lactose, mannitol, mannitol, sorbitol, maltitol, trehalose, calcium carbonate, magnesium carbonate, and silica.

5. The formulation according to any one of claims **1** or claim **4,** wherein the second coating composition represents from 2% to 30% relative to the total weight of the core-shell particulate formulation.

6. The formulation according to any one of claims **1** to **5,** further comprising a final coating in an amount ranging from 2 to 20% relative to the total weight of the core-shell particulate formulation, said final coating comprising:
- Polyvinyl alcohol in association with talc, titanium dioxide, glyceryl mono and dicaprylocaprate and sodium lauryl sulfate;
- polyvinyl alcohol-polyethylene glycol graft copolymer, polyvinyl alcohol, and silicone dioxide;
- Hypromellose and talc; or
- a mixture thereof.

7. The formulation according to any one of claims **1** to **6,** wherein the core consists of a pharmaceutically acceptable inert core, coated with a first coating composition comprising, in weight relative to the total weight of the coated core:
- from 10% to 70% w/w of probenecid;
- from 20% to 40% w/w of hydroxypropylcellulose;
- from 1% to 3% w/w of a surfactant; and
- optionally an anti-foaming agent.

8. The formulation according to any one of claims **1** to **7,** wherein the inert core consists of microspheres consisting of at least one pharmaceutically acceptable excipient presenting an average diameter ranging from 100 µm to 5 mm, preferably the inert core consists of microcrystalline cellulose microspheres presenting an average diameter ranging from 100 µm to 800 µm, more preferably from 300 to 400 µm, the average diameter being determined with the sieving method.

9. The formulation according to any one of claims **7** or **8,** wherein the surfactant is a non-ionic surfactant, preferably the surfactant is polyoxyethylene (20) sorbitan monooleate.

10. The formulation according to any one of claims **3** to **9,** wherein the first coating composition represents from 50% to 80% in weight relative to the total dry weight of the coated core.

11. The formulation according to any one of claims **1** to **10,** wherein the core further comprises a seal-coating composition in an amount ranging from 2% to 20% in weight relative to the coated cores, said seal-coating composition comprising:
- polyvinyl alcohol in association with talc, titanium dioxide, glyceryl mono and dicaprylocaprate and sodium lauryl sulfate;
- polyvinyl alcohol-polyethylene glycol graft copolymer, polyvinyl alcohol, and silicone dioxide;
- Hypromellose and talc; or
- A mixture thereof.

12. The formulation according to any one of claims **1** to **11,** for use as a drug

13. The formulation according to any one of claims **1** to **11,** for use in the treatment of epilepsy.

14. A process for preparing the core-shell particulate formulation according to any one of claims **1** to **11,** comprising the steps of:
a) Supplying a composition of cores comprising probenecid in an amount ranging from 10% to 70% in weight relative to the total weight of the core, in association with at least one pharmaceutically acceptable excipient,
b) Coating the cores with a shell coating composition comprising triethyl citrate, a mineral charge, polyvinyl acetate, polyvinylpyrrolidone and sodium lauryl sulfate, **characterized in that** the weight ratio of polyvinyl acetate on polyvinylpyrrolidone ranges from 1.5 to 4, preferably by spray drying, leading to a core-shell particulate formulation,
c) Optionally further coating with a final coating as described in claim 6;
d) Recovering the core-shell particulate formulation obtained in any one of steps b) or c).

15. The process according to claim **14,** wherein step a) comprises:
a1) Supplying a composition of inert core consisting of at least one pharmaceutically acceptable excipient;
a2) Coating the microcrystalline cellulose microspheres, preferably by spray drying, with a first coating composition, thereby supplying coated cores of microcrystalline cellulose, wherein the first coating composition comprises, in weight relative to the total weight of the coated cores:
- from 10% to 70% w/w of probenecid;
- from 20% to 40% w/w of hydroxypropylcellulose;
- from 1% to 3% w/w of a surfactant; and
- optionally an anti-foaming agent;
a3) optionally, further coating the coated cores of microcrystalline cellulose with a seal coating composition as described in claim 11,
a4) recovering the particulate formulation obtained in step a2) or in step a3).
